# EUROPEAN PATENT SPECIFICATION

(11) **EP 2 441 479 B1**
(45) Date of publication and mention of the grant of the patent: **02.05.2018**
(21) Application number: 10785916.7
(22) Date of filing: 04.06.2010
(51) Int. Cl.: A61M 1/00, A61M 27/00, A61B 17/32, A61M 25/00

(54) **SURGICAL SUCTION NOZZLE**
CHIRURGISCHE SAUGDÜSE
BUSE D'ASPIRATION CHIRURGICALE

(30) Priority: 10.06.2009 JP 2009139018
(43) Date of publication of application: 18.04.2012
(73) Proprietor: Tani, Tohru, Shiga 525-0057 (JP)
(72) Inventor: Tani, Tohru, Shiga 525-0057 (JP)
(74) Representative: Forstmeyer, Dietmar
(86) International application number: PCT/JP2010/003734
(87) International publication number: WO 2010/143384

(56) References cited:
- WO-A1-00/11394
- JP-A- 6 285 155
- JP-A- 2001 252 349
- JP-U- H0 588 552
- JP-U- H01 138 449
- NL-C2- 1 020 597
- US-A- 4 815 462
- US-A- 6 086 587
- US-A1- 2004 122 447
- US-B1- 6 491 692

## Description

### Technical Field

The present invention relates to a surgical suction cannula that sucks and removes blood, exuding body fluid, exfoliated small tissues and cleansing liquid during a surgical operation.

### Background Art

The suction of blood, cleansing liquid, and body fluid during a surgical operation is a very maneuver for ensuring a surgical view and is involved in the safety and precision of the surgical operation. In general, a surgical suction tube includes an elongated cylindrical suction tube connected to a suction device of central piping and sucks blood flowing out to a surgical site and cleansing liquid by a cannula at a tip end of the suction tube. There are disposable or reusable surgical suction tubes, and most of the surgical suction tubes have a tip opening that is several millimeters in size at a tip end. Further, the surgical suction tubes have a hole on a side wall, or have an outside cap with a side hole to prevent suction of tissue. Thus, the surgical suction tubes are devised so as to suck liquid components effectively.

The same cannula may be provided with a water injection valve at some midpoint thereof so as to have a cleansing function or may also have electrical coagulation ability.

Further, in a brain surgery, the suction is conducted through a small cotton sheet so that a brain tissue is not damaged by the suction. In a laparoscopic surgical operation, the suction needs to be conducted with a gauze cut to a small size inserted in some cases. In the case of bleeding in which blood blows out from a blood vessel, a bleeding point becomes invisible immediately, which makes it necessary to conduct astriction at the bleeding point simultaneously with the suction. However, the conventional suction accelerates the bleeding, which may even enlarge a bleeding hole.

If a suction port is limited to the tip opening, a soft organ, a greater omentum, and a blood clot adsorb to the tip opening during the suction, which hinders the suction. Therefore, in an abdominal surgical operation, the suction is conducted with an outer cylinder having a large number of minute holes fitted to a side wall (Patent Literature 1). However, the greater omentum and the like are sucked by the minute holes to close the holes, which makes it impossible to conduct effective suction and may also damage a sucked tissue. Further, when the side hole is opened, air enters from the side, and hence, a small amount of remaining fluid and blood cannot be sucked. Then, there is a demand for means for removing liquid components such as a small amount of remaining accumulated fluid and a small amount of blood covering the surface of an organ effectively without damaging the organ.

In particular, the laparoscopic surgical operation is performed with carbon dioxide stored in an abdominal cavity. If the side hole is opened in the suction tube, a great amount of pressurized carbon dioxide is sucked from the side, which eliminates the effect of gas enlarging the abdominal cavity. As a result, a view cannot be obtained. Further, in the laparoscopic surgical operation, it is difficult to remove the entire blood that has been flown out by allowing a gauze to absorb the blood. Further, in the laparoscopic surgical operation, it is necessary to finish the surgical operation without replacing a cannula frequently. Thus, there is a demand for means for removing liquid components such as a small amount of remaining blood and a small amount of bloody body fluid remaining on the surface of an organ effectively without damaging the organ and sucking pressurized gas in the laparoscopic surgical operation.

In the case where there is further bleeding in a surgical site, it is necessary to ensure a view enabling a hemostatic site to be treated by pressurizing a bleeding site while sucking blood to control the bleeding. There is a demand for means having a suction effect simultaneously with bleeding control.

### Citation List

### Patent Literature

Patent Literature 1: Japanese UtilityModelApplication Laid-open No. Hei 7-22743.
US6086587A, US6491692B1, US2004/122447A1, and WO00/11394A1 are relevant prior art.

### Summary of Invention

### Technical Problem

The present invention provides a surgical suction cannula capable of sucking a liquid component such as a small amount of blood to present a bleeding site without damaging an organ while keeping an ability to suck a large clot such as a blood clot.

### Solution to Problem

The inventor of the present invention has intensively studied so as to solve the above-mentioned problem, and found that the problem can be solved by a cannula as defined in the appended claims.

### Advantageous Effects of Invention

The surgical cannula of the present invention is capable of collecting and sucking liquid components such as a small amount of blood and body fluid through a capillary action without choking the orifice while keeping the ability to suck a large clot such as a blood clot in an open surgical operation or laparoscopic surgical operation. Further, the surgical cannula is capable of sweeping as in a broom. Therefore, even a blood clot on the surface of an organ can be removed as if the blood clot was wiped away while the surface is being cleansed so that the original color of the organ can be recognized.

Further, even when the surgical cannula is pressed against an organ, the suction structure interposed between the suction port and the surface of the organ hinders the adsorption, and thus, the effective suction of fluid can be conducted smoothly and rapidly.

Further, the bleeding can be controlled by pressurizing a bleeding site with the suction structure. Simultaneously, blood can be sucked so that the blood is not accumulated, and hence, a bleeding point becomes clear and the effective hemostasis can be conducted.

In the suction in brain surgery, it is a contraindication to damage a brain tissue. The suction cannula of the invention of the present application is capable of collecting blood and cleansing liquid in the suction structure through a capillary action and an operation as in a broom and conducting the suction without bringing the suction cannula into contact with the brain tissue, and is further capable of controlling a bleeding site. Therefore, the surgical suction cannula of the invention of the present application is particularly effective in the suction in the brain surgery.

### Brief Description of Drawings

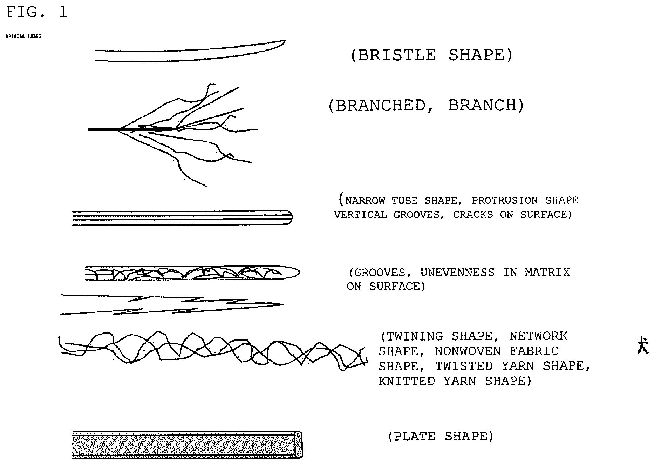

[FIG. 1] A view illustrating shapes and states of a surface of a bristle or a protrusion constituting a suction structure according to the present invention.
[FIG. 2] A perspective view of a tip end of a surgical suction cannula according to Example 1.
[FIG. 3] A perspective view of a tip end of a surgical suction cannula according to Example 2.
[FIG. 4] A perspective view of a tip end of a surgical suction cannula according to Example 3.

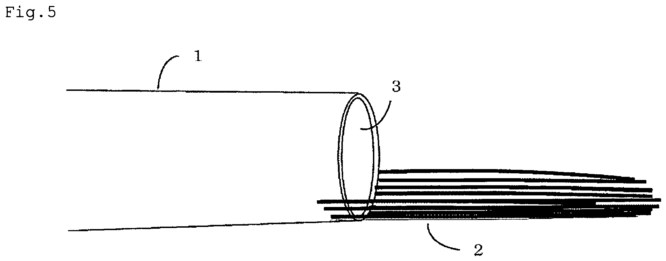

[FIG. 5] A perspective view of a tip end of a surgical suction cannula according to one embodiment of the present invention.

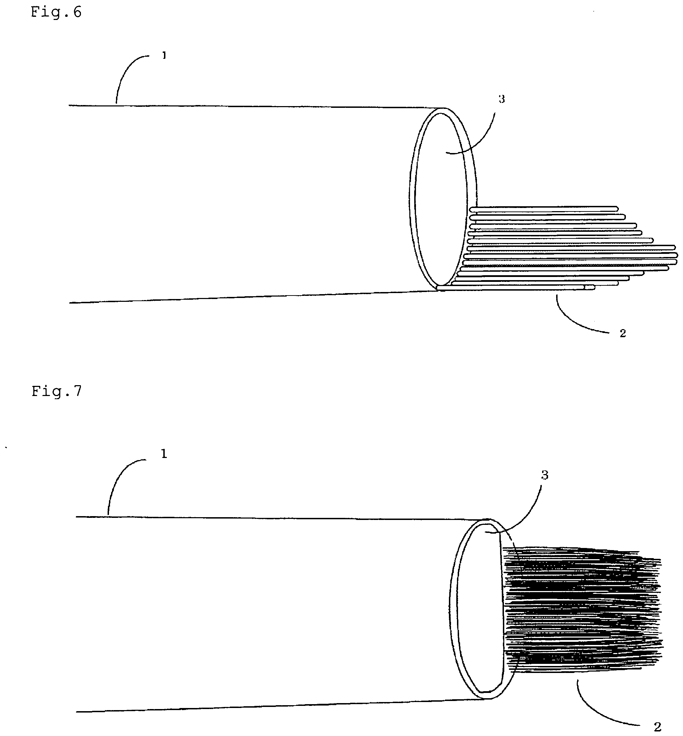

[FIG. 6] A perspective view of a tip end of a surgical suction cannula according to another embodiment of the present invention.
[FIG. 7] A perspective view of a tip end of a surgical suction cannula according to a further embodiment of the present invention.

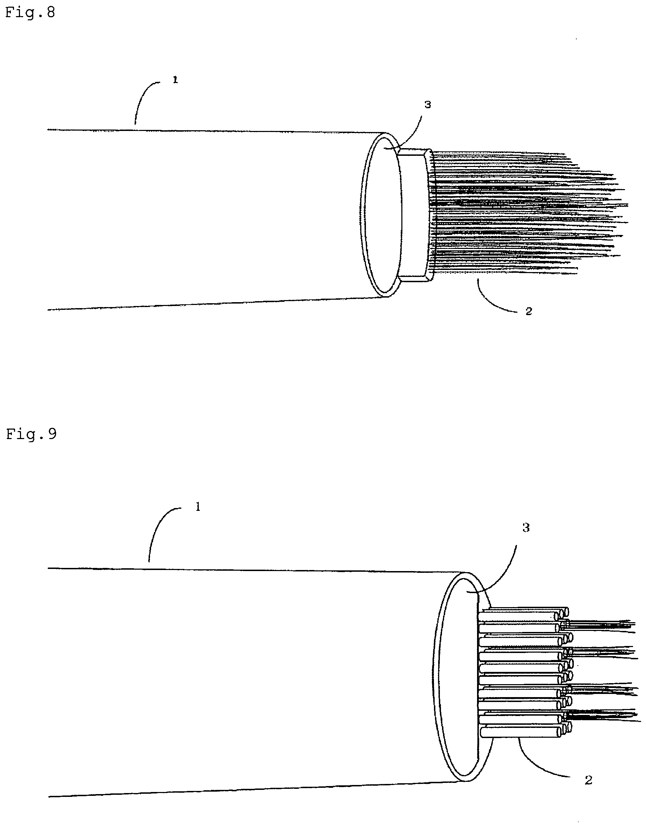

[FIG. 8] A perspective view of a tip end of a surgical suction cannula according to a still further embodiment of the present invention.
[FIG. 9] A perspective view of a tip end of a surgical suction cannula according to a yet further embodiment of the present invention.

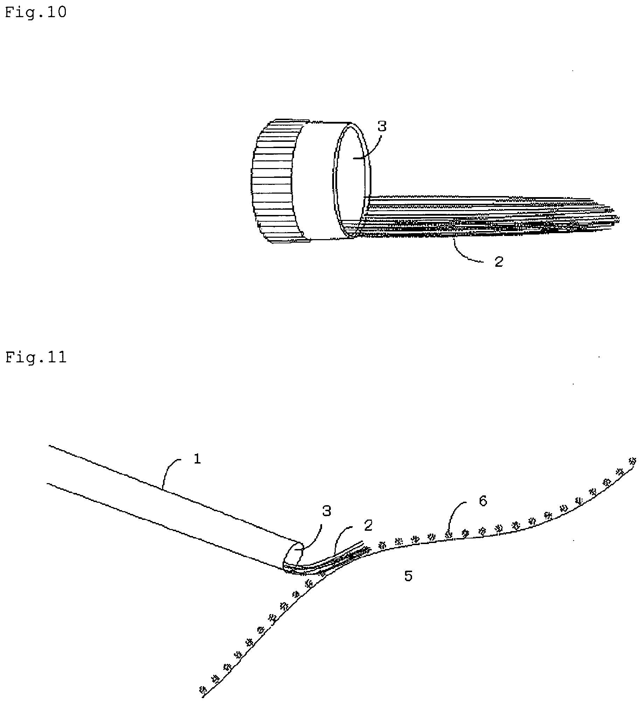

[FIG. 10] A perspective view of a tip end unit for a surgical suction cannula according to one embodiment of the present invention.
[FIG. 11] A use example of the surgical suction cannula of the present invention, illustrating that liquid components such as a small amount of blood and body fluid remaining on a surface of an organ can also be sucked.

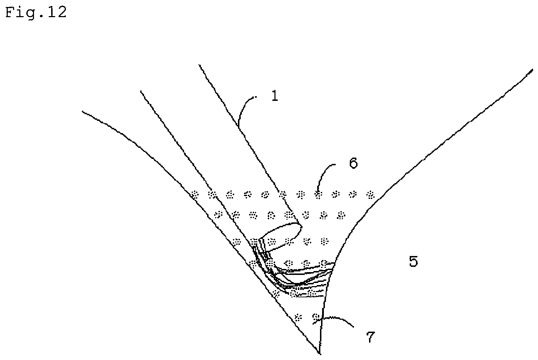

[FIG. 12] A use example of the surgical suction cannula of the present invention, illustrating that liquid components such as a small amount of blood and body fluid remaining at a narrow site can be sucked completely.

### Description of Embodiments

The invention of the present application relates to a surgical suction cannula, including: a tip opening capable of sucking a clot such as a blood clot; and a flexible suction structure extending in an axial front direction of a cannula from the tip opening.

Herein, the tip opening refers to an opening of a tip end portion of the cannula that sucks blood, body fluid, and cleansing liquid caused during a surgical operation in the surgical suction cannula, and the axial front direction of the cannula from the tip opening refers to a further front direction from the vicinity of the tip opening. The surgical suction cannula of the invention of the present application has a flexible suction structure that extends in the axial front direction of the cannula from the tip opening, and the tip opening keeps an ability to suck a clot such as a blood clot.

The suction structure according to the invention of the present application is a flexible structure that is formed of a plurality of bristles and/or protrusions. Examples of the suction structure include a bristle structure formed of a plurality of bristles, a protrusion structure formed of a plurality of flexible protrusions, and a structure formed of a plurality of bristles and a plurality of protrusions.

The suction structure used in the present invention causes a capillary action. The bristles and protrusions are not so hard as to damage an organ, have elasticity and resilience, return to the original shapes even after being pressurized, are not too soft, and have elasticity and hardness to such a degree that the bristles and protrusions are not sucked by a suction port. It is preferred that the suction structure have a function of alleviating the contact between a tissue and a cannula opening in suction during a surgical operation and simultaneously achieve sufficient suction. The suction structure can suck liquid components such as a small amount of blood through a capillary action of the suction structure and can simultaneously perform an operation of sweeping as in a broom. On the other hand, when the suction structure is dense and has elasticity, the suction structure can control the bleeding by pressurizing a point at a time of bleeding, and can simultaneously suck blood in such a manner that the blood is not accumulated. Further, even if the tip opening is pressed against the vicinity of an organ, the suction structure is interposed between the suction port and the surface of the organ to prevent the choke between the organ and the cannula or damage to the organ due to the choke by suction.

It is preferred that the suction structure of the cannula of the present invention be shaped so as not to damage an organ, have softness and elasticity, and have a tip end which is thin and bent smoothly. Further, in order to allow the cannula to proceed to a target site in the laparoscopic surgical operation, it is preferred that the tip end of the suction structure be thin as a whole.

The bristle and protrusion may have one root and be branched at a periphery. Further, the bristle and protrusion may have a narrow tube shape, a twining shape, a network shape, a twisted yarn shape, a knitted yarn shape, a nonwoven fabric shape, or a plate shape.

Further, although the surface of the bristle and the protrusion may be smooth, the surface of the bristle and the protrusion may be provided with unevenness or grooves so as to cause a capillary action easily. For example, the bristle and the protrusion may be provided with vertical grooves, cracks, or grooves in a matrix.

It is preferred that the shape of the bristle or the protrusion close to a tip end thereof be tapered. Further, it is suitable that the tip end be rounded.

The cross-section of the bristle may be circular or non-circular without any limit, and the diameter of the cross-section in the case where the bristle is circular is preferably 0.001 to 1 mm, more preferably 0.005 to 0.5 mm, still more preferably 0.01 to 0.2 mm, most preferably 0.05 to 0.15 mm. The suitable thickness of the bristle varies depending upon an organ for which the bristle is used and a purpose of use.

The cross-section of the protrusion may be circular or non-circular without any limit, and the diameter of the cross-section in the case where the protrusion is circular is preferably 0.1 to 1 mm, more preferably 0. 3 to 0. 8 mm. In the case where the protrusion is oval, the preferred long diameter thereof is 0.1 to 1 mm, and the preferred short diameter thereof is 0.001 to 0.5 mm. The suitable thickness of the protrusion varies depending upon a material to be used, an organ for which the protrusion is used, and a purpose of use.

In the case of a thick bristle or protrusion, it is preferred that the bristle or protrusion be devised so as to be provided with unevenness or grooves on the surface or so as to have a knitted yarn shape or a twisted yarn shape.

Regarding the length of the suction structure of the cannula of the present invention, the length from the tip opening to the tip end of the suction structure is preferably 0.2 to 10 times, more preferably 0. 5 to 5 times the diameter of the tip opening. That length is preferably 2 mm to 30 mm, more preferably 2 mm to 20 mm, still more preferably 5 mm to 15 mm. If the length is smaller than the length that is 0.2 times the diameter of the tip opening, the effect of protecting a tissue and the like is insufficient, and the effect of removing a small amount of liquid components in a narrow portion is insufficient. If the length is larger than the length that is 10 times the diameter of the tip opening, the effect of suction is degraded, with the result that the suction structure hinders the surgical operation. Although the small length of the suction structure is unsuitable for the protection of a tissue, the suction structure has an advantage of keeping the suction force, and the suitable length and hardness vary depending upon an organ for which the suction structure is used and a purpose of use.

It is preferred that the number and density of bristles or protrusions constituting the suction structure of the cannula of the present invention be set to form such an assembly as to exhibit the sufficient effect of a capillary action. Further, it is preferred that the number and density of bristles or protrusions be set to form such an assembly as to prevent the adhesion between an organ and the cannula or damage to the organ due to the adhesion by suction. Further, it is preferred that the number and density of bristles or protrusions be set to form such an assembly as to control bleeding.

The surgical suction cannula of the invention of the present application has a tip opening capable of sucking a clot such as a blood clot. Having a tip opening capable of sucking a clot such as a blood clot refers to having an opening with such a size as to suck not only liquid blood, cleansing liquid, and the like, but also a clot such as a blood clot.

In the surgical suction cannula of a preferred embodiment of the invention of the present application, the suction structure is arranged on a tip opening tube wall of the suction cannula. Examples of the arrangement position include an inner side, a tip end, and an outer side of the tip opening tube wall.

In the surgical suction cannula of one embodiment of the invention of the present application, the suction structure is arranged along the tip opening tube wall. The arrangement position and arrangement method are not limited. For example, the suction structure may be arranged at the tip end of the opening tube wall, or may be arranged along the outer side or inner side of the tube wall.

The suction structure is arranged along a part or all of the tip opening tube wall of the cannula. Depending upon the purpose, the bristles or protrusions are arranged along 5% to 100%, preferably 10% to 100%, more preferably 15% to 100% of the circumference of the opening tube wall. Depending upon an organ and a purpose of use, the bristles or protrusions are arranged along preferably 10% to 80%, more preferably 15% to 60%, still more preferably 20% to 50% of the circumference.

The suitable arrangement ratio of the suction structure varies depending upon an organ and a purpose of use. In the case of brain surgery in which it is a contraindication to damage an organ by suction and a suction amount is small, the suction structure may be arranged around the opening. In the case where it is required to suck a great amount of liquid components, the suction structure may be arranged so as to inhibit the adsorption with respect to an organ even if a capillary action may not be realized. In the case where the suction structure is arranged partially, it is suitable that the suction structure be arranged along the opening tube wall on a lower side (side on which there is a possibility of the contact with a tissue and the like) of the cannula. If the suction structure is arranged along 5% or less of the circumference, it becomes difficult to achieve the effect of target suction, the effect of protecting a tissue, and the effect of hemostasis.

In the case where the bristles or protrusions are arranged along the tip opening tube wall, the bristles or protrusions may be arranged one by one, or may be arranged on the basis of a plurality of the bristles or protrusions. Further, the bristles or protrusions may be arranged in one, two, three, or more lines.

In another embodiment of the present invention, the suction structure formed by packing a plurality of bristles and/or protrusions is arranged in an inner portion of the cannula, an outer portion thereof, or at a tip end of the tube wall. In the case of any arrangement, the suction structure is suitably arranged on a lower side (side on which there is a possibility of the contact with a tissue and the like) of the cannula.

In the case where the suction structure formed by packing a plurality of bristles and/or protrusions is present in an inner portion of the tip opening, the ratio of the cross-sectional area of the suction structure with respect to the cross-sectional area of the tip opening of the cannula is preferably 5% to 60%, more preferably 10% to 60%, still more preferably 15% to 45%, yet more preferably 20% to 40%. By setting the cross-sectional area of the suction structure to be 60% or less, an opening can be ensured, which is capable of sucking a clot such as a blood clot.

In the case where the suction structure formed by packing a plurality of bristles and/or protrusions is arranged in an outer portion of the cannula or arranged at a tip end of the tube wall, the tip opening is ensured as an opening. It is preferred that the amount of the suction structure be equal to that in the case where the suction structure is arranged in an inner portion of the cannula.

In the case where the suction structure has a configuration in which a plurality of bristles and/or protrusions are packed, the specific number of the bristles and/or protrusions is about 10 to 500. The number is changed depending upon the thickness and shape of the bristles and/or protrusions, a purpose, and an organ in a surgical site.

The material for the cannula of the present invention is not particularly limited, and a generally used one may be used. For example, a metal material such as stainless steel, a titanium alloy, duralumin, or aluminum or a resin material such as a nylon resin, an ABS resin, a polycarbonate resin, or saturated propylene may be used. A composite material of a metal material and a resin material may also be used.

It is preferred that the material for bristles or protrusions of the suction structure of the present invention be soft and do not damage an organ. However, there is no particular limitation to the material, as long as the material has flexibility and elasticity, bends smoothly, and has resilience. For example, natural fibers, natural hair, artificial fibers, artificial hair, plastic, nylon, vinylon, saturated propylene, and the like can be used. Those which are generally used for a toothbrush may be used.

In order to prevent the suction structure from coming off from the cannula, the material for the cannula may be set to be the same as that for the suction structure. It is preferred that the suction structure can be molded integrally with the tube wall of the cannula.

The suction structure can be formed of a thin hollow yarn. In the case where the suction structure is arranged in an inner portion of the cannula, liquid components such as blood and body fluid can be sucked by the suction through the cannula, and hence, the hollow yarn may or may not be connected to a central suction cleansing device. The effect of sucking liquid components is further enhanced by the ability to suck through the tip opening and the hollow yarn. It is preferred that the hollow yarn be connected to the central suction cleansing device, and in this case, it is preferred that the suction be divided into the suction by the suction structure and the suction by the tip opening. By dividing the suction, the operation and adjustment of the suction and cleansing can be performed individually in the hollow yarn portion and the tip opening.

It is preferred that the surgical suction cannula of the present invention do not have a hole on the side wall of the cannula for sucking a small amount of fluid. If the side hole is opened, air enters from the side, which makes it difficult to suck a small amount of remaining fluid and blood. Particularly in the laparoscopic surgical operation, if the side hole is opened, air and the like enter from the side to eliminate the effect of enlarging an abdominal cavity by the gas pressurization.

A thick suction tube having a hole on a side wall is preferred for cleansing (about 1 to 10 L are used) during the abdominal surgical operation. In this case, only the effect that the suction tube does not adhere to a tissue suffices, and preferably, the suction structure may be provided outside of the side hole.

The suction structure is fixed to the tip opening of the cannula or the periphery thereof. Although there is no particular limitation to the fixing method, the suction structure can be fixed by weld joining, adhesion joining, integral molding, bondage, or the like.

The suction structure, or the tip opening or the periphery thereof to which the suction structure is fixed may be removable from the suction cannula body.

Because it is difficult to cleanse and disinfect the suction structure of the invention of the present application after the use, it is preferred that the suction structure be disposable. If the suction cannula body is reusable, only the suction structure may be disposable. The tip opening of the suction cannula may also be disposable. Further, the tip opening and the suction structure may be molded integrally.

At a time of use, the suction structure is connected to the suction cannula body. It is preferred that the suction structure be attached to the suction cannula by one-touch attachment through a cassette-shaped attachment unit. The suction structure may be attached to the suction cannula through a screw, fitting, an elastic band, or the like. The suction cannula itself may be disposable.

The present invention also includes a surgical suction cannula including a reusable suction cannula and a disposable suction structure attached removably to the tip end of the suction cannula.

The cannula of the present invention may have an outer cylinder. The outer cylinder is suitable for allowing the cannula of the present invention to proceed to a target site in the laparoscopic surgical operation. Further, the cannula of the present invention may have functions other than the suction. Examples of the functions include a cleansing function and a coagulation ability with an electromagnetic wave. For example, in the case where the surgical suction cannula of the invention of the present application is connected to cleansing means, the surgical suction cannula has a cleansing function. In the case where the surgical suction cannula is connected to coagulation incision means, the coagulation incision means can be extended to a surgical site from an inner portion or an outer portion of the cannula of the present invention.

Further, the surgical suction cannula of the present invention can be used together with the coagulation incision means and/or cleansing means for a living tissue. Further, the present invention provides a surgical treatment tool in which the surgical suction cannula, and the coagulation incision means and/or the cleansing means are integrated. Examples of the tool include a surgical treatment tool in which the coagulation incision means and/or the cleansing means are provided outside of the suction cannula of the present invention, and a surgical treatment tool including the suction cannula of the present invention, and the coagulation incision means and/or the cleansing means in the outer cylinder. The treatment tools provided with these means enable a surgical operation to be performed precisely and easily. An example of the coagulation incision means is means for cutting a tissue after coagulating the tissue with an electromagnetic wave.

The present invention further relates to a surgical suction cannula tip end unit including a suction structure connectable to the surgical suction cannula body. The surgical suction cannula tip end unit includes a cannula connecting portion and a suction structure. The surgical suction cannula tip end unit may further include a surgical suction cannula tip opening. It is preferred that the tip opening and the suction structure be made of the same material or be molded integrally.

It is preferred that the suction cannula tip end unit be disposable. When the suction cannula tip end unit of the present invention is connected to the surgical suction cannula body, the suction cannula tip end unit includes a surgical structure capable of forming the suction cannula of the present invention.

The tip end unit is connected to the surgical suction cannula body through a screw, fitting, an elastic band, or the like. It is preferred that the tip end unit be connected to the surgical suction cannula body by one-touch attachment through a cassette-shaped attachment unit or an elastic band.

Hereinafter, examples of the present invention are described with reference to the drawings.

### Example 1

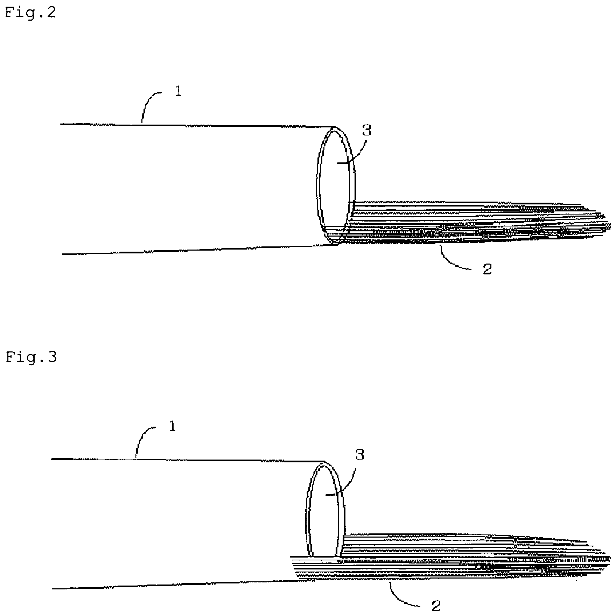

FIG. 2 illustrates a surgical suction cannula of this example. The surgical suction cannula of this example includes a cylindrical suction cannula body portion 1 and a suction structure 2 formed of bristles arranged at the tip end of the suction cannula body portion 1. The suction structure was integrally molded with a 30% portion of the circumference of a tip opening 3 of a cannula. The length from the opening of the cannula to the tip end of the suction structure is 2.2 times (1.1 cm) the outer diameter of the tip opening. The tip end portion of the suction structure is thinned as a whole. The bristles are made of a nylon material, and the thickness thereof is 100 µm in diameter. The vicinity of the tip end of the bristles is tapered, and the tip end portion has a semi-spherical shape. When the cannula having the suction structure was lightly pressed against the surface of an organ of a dog and liquid components were sucked as if the liquid components were swept, a small amount of the liquid components on the surface of the organ was sucked without the surface of the organ adhering to the cannula. Further, when the cannula was pressed against the small amount of the liquid components remaining in a small dent and the liquid components were sucked, the small amount of the liquid components was sucked without the cannula adhering to the organ.

### Example 2

FIG. 3 illustrates a surgical suction cannula of this example. The surgical suction cannula has the same shape and material as those of the suction cannula of Example 1, except that the surgical suction cannula is obtained by weld-joining the suction structure to an outer portion of the tip opening tube wall of the cannula. When the effect of the cannula having this suction structure was checked in the same way as in Example 1, the satisfactory results were obtained in the same way as in Example 1.

### Example 3

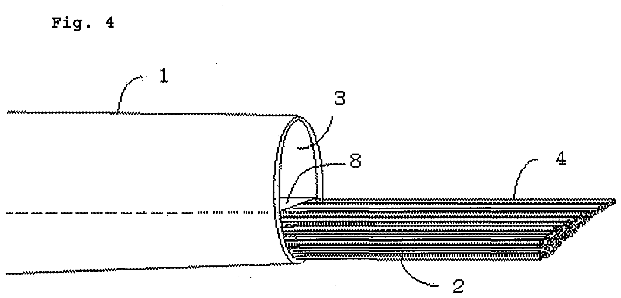

FIG. 4 illustrates a surgical suction cannula of this example. The surgical suction cannula of this example includes a cylindrical suction cannula body portion 1. A suction structure 2 is present in a lower portion inside the suction cannula body and the suction structure extends forward from inside of a tip opening 3. The suction structure is formed of a hollow yarn 4, and the suction is divided into the suction by the tip opening and the suction by the hollow yarn. A suction passage by the tip opening and the suction structure formed of the hollow yarn are separated from each other by a partition wall 8 inside the cannula. The hollow yarn is connected to a central suction cleansing device and is capable of sucking liquid components such as blood and body fluid independently from the tip opening. If the hollow yarn is stuck, the hollow yarn is capable of sucking the liquid components after cleansing. The hollow yarn is fixed to an inner side of the suction cannula body through adhesion joining. The hollow yarn occupies 35% of the cross-sectional area of the tip opening. The length from the tip opening to the tip end of the hollow yarn is twice (1.0 cm) the outer diameter of the tip opening. The bristles are made of a nylon material, and the thickness thereof is 500 µm in diameter and 400 µm in inner diameter. When the cannula having the suction structure was pressed against the surface of an organ of a dog and liquid components were sucked, the liquid components were sucked without the surface of the organ adhering to the cannula. Further, when the small amount of the liquid components remaining in a small dent was sucked, the small amount of the liquid components was sucked without damaging the organ.

### Reference Signs List

- 1: cylindrical suction cannula body
- 2: suction structure
- 3: tip opening
- 4: hollow yarn
- 5: organ
- 6: liquid component such as blood and body fluid
- 7: narrow portion in surgical site
- 8: partition wall inside cannula

## Claims

1. A surgical suction cannula, comprising:
a tip opening (3) capable of sucking a clot such as a blood clot; and
a flexible suction structure (2) comprising a bristle structure including a plurality of bristles extending in an axial front direction of a cannula from the tip opening (3), wherein the flexible suction structure (2) is formed of elastic bristles, and has a tip end which is bent smoothly,
wherein the suction structure (2) can perform an operation of sweeping as in a broom,
and wherein the suction structure (2) hinders the adsorption to the surface of an organ, **characterised in that** the suction structure causes a capillary action.

2. A surgical suction cannula according to claim 1, wherein the diameter of the cross-section of the bristle is 0.001 to 1 mm.

3. A surgical suction cannula according to claim 1, wherein the cannula is used for sucking a blood clot, blood, exuding body fluid, and cleansing liquid.

4. A surgical suction cannula according to claim 1, wherein the flexible suction structure (2) is arranged at a tip opening tube wall of the surgical suction cannula.

5. A surgical suction cannula according to claim 1 or 2, wherein the flexible suction structure (2) is arranged along the tip opening tube wall.

6. A surgical suction cannula according to any one of claims 1 to 5, wherein the flexible suction structure (2) comprises a suction structure formed by packing a plurality of bristles, and in a cross-section of the tip opening of the surgical suction cannula, a ratio of a cross-sectional area of the suction structure to a cross-sectional area of the tip opening of the cannula is 5% to 60%.

7. A surgical suction cannula according to any one of claims 1 to 6, wherein the flexible suction structure (2) comprises a disposable suction structure connectable to and removable from a suction cannula body (1).

8. A surgical suction cannula according to any one of claims 1 to 7, wherein a length from the tip opening (3) to a tip end of the flexible suction structure (2) is 0.2 times to 10 times a diameter of the tip opening (3).

9. A surgical suction cannula tip end unit, comprising a suction structure (2), the surgical suction cannula tip end unit being capable of forming the surgical suction cannula according to claims 1 to 8 by being connected to the suction cannula body (1).

10. A surgical suction cannula tip end unit according to claim 9, comprising a suction structure (2) and a cannula tip opening (3),
wherein the suction structure (2) is arranged at a cannula tip opening tube wall.

11. A surgical treatment tool, comprising:
the surgical suction cannula according to any one of claims 1 to 8; and
coagulation incision means and/or cleansing means.

## Patentansprüche

1. Chirurgische Absaugkanüle, umfassend:
eine Spitzenöffnung (3), die in der Lage ist, ein Gerinnsel wie ein Blutgerinnsel abzusaugen; und
eine flexible Saugstruktur (2), die eine Borstenstruktur mit einer Mehrzahl von Borsten umfasst, die sich in einer axialen vorderen Richtung einer Kanüle von der Spitzenöffnung (3) aus erstreckt,
wobei die flexible Saugstruktur (2) aus elastischen Borsten gebildet ist und ein Spitzenende aufweist, das sanft gebogen wird,
wobei die Saugstruktur (2) einen Kehrvorgang wie bei einem Besen ausführen kann,
und wobei die Saugstruktur (2) die Adsorption an die Oberfläche eines Organs verhindert, **dadurch gekennzeichnet, dass** die Saugstruktur eine Kapillarwirkung verursacht.

2. Chirurgische Absaugkanüle nach Anspruch 1, wobei der Durchmesser des Querschnitts der Borste 0,001 bis 1 mm beträgt.

3. Chirurgische Absaugkanüle nach Anspruch 1, wobei die Kanüle dazu verwendet wird, um ein Blutgerinnsel, Blut, exsudierende Körperflüssigkeit und Reinigungsflüssigkeit abzusaugen.

4. Chirurgische Absaugkanüle nach Anspruch 1, wobei die flexible Saugstruktur (2) an einer Rohrwand der Spitzenöffnung der chirurgischen Absaugkanüle angeordnet ist.

5. Chirurgische Absaugkanüle nach Anspruch 1 oder 2, wobei die flexible Saugstruktur (2) entlang der Rohrwand der Spitzenöffnung angeordnet ist.

6. Chirurgische Absaugkanüle nach einem der Ansprüche 1 bis 5, wobei die flexible Saugstruktur (2) eine Saugstruktur umfasst, die durch das Packen einer Mehrzahl von Borsten und in einem Querschnitt der Spitzenöffnung der chirurgischen Absaugkanüle gebildet ist, wobei ein Verhältnis einer Querschnittsfläche der Saugstruktur zu einer Querschnittsfläche der Spitzenöffnung der Kanüle 5% bis 60% beträgt.

7. Chirurgische Absaugkanüle nach einem der Ansprüche 1 bis 6, wobei die flexible Saugstruktur (2) eine wegwerfbare Saugstruktur aufweist, die mit einem Saugkanülenkörper (1) verbindbar und von diesem abnehmbar ist.

8. Chirurgische Absaugkanüle nach einem der Ansprüche 1 bis 7, wobei eine Länge von der Spitzenöffnung (3) zu einem Spitzenende der flexiblen Saugstruktur (2) das 0,2-fache bis 10-fache eines Durchmessers der Spitzenöffnung (3) beträgt.

9. Spitzenendeinheit einer chirurgischen Absaugkanüle, umfassend eine Saugstruktur (2), wobei die Spitzenendeinheit der chirurgischen Absaugkanüle die chirurgische Absaugkanüle der Ansprüche 1 bis 8 bilden kann, indem sie mit dem Absaugkanülenkörper (1) verbunden ist.

10. Spitzenendeinheit einer chirurgischen Absaugkanüle nach Anspruch 9, umfassend eine Saugstruktur (2) und eine Kanülenspitzenöffnung (3),
wobei die Saugstruktur (2) an einer Rohrwand der Kanülenspitzenöffnung angeordnet ist.

11. Chirurgisches Behandlungswerkzeug, umfassend:
die chirurgische Absaugkanüle nach einem der Ansprüche 1 bis 8; und
Koagulationsinzisionsmittel und/oder Reinigungsmittel.

## Revendications

1. Canule d'aspiration chirurgicale, comprenant :
une ouverture de pointe (3) capable d'aspirer un caillot tel qu'un caillot de sang ; et
une structure d'aspiration souple (2) comprenant une structure à poils comprenant une pluralité de poils s'étendant dans une direction axiale avant d'une canule à partir de l'ouverture de pointe (3), dans laquelle la structure d'aspiration souple (2) est formée à partir de poils élastiques, et a une extrémité de pointe qui est pliée en douceur,
dans laquelle la structure d'aspiration (2) peut réaliser une opération de balayage comme un balai,
et dans laquelle la structure d'aspiration (2) empêche l'adsorption à la surface d'un organe, **caractérisée en ce que** la structure d'aspiration provoque une action capillaire.

2. Canule d'aspiration chirurgicale selon la revendication 1, dans laquelle le diamètre de la section transversale du poil est de 0,001 à 1 mm.

3. Canule d'aspiration chirurgicale selon la revendication 1, dans laquelle la canule est utilisée pour aspirer un caillot de sang, du sang, du fluide corporel d'exsudat, et du liquide nettoyant.

4. Canule d'aspiration chirurgicale selon la revendication 1, dans laquelle la structure d'aspiration souple (2) est agencée au niveau d'une paroi tubulaire d'ouverture de pointe de la canule d'aspiration chirurgicale.

5. Canule d'aspiration chirurgicale selon la revendication 1 ou 2, dans laquelle la structure d'aspiration souple (2) est agencée le long de la paroi tubulaire d'ouverture de pointe.

6. Canule d'aspiration chirurgicale selon l'une quelconque des revendications 1 à 5, dans laquelle la structure d'aspiration souple (2) comprend une structure d'aspiration formée en compactant une pluralité de poils, et dans une section transversale de l'ouverture de pointe de canule d'aspiration chirurgicale, un rapport d'une surface transversale de la structure d'aspiration sur une surface transversale de l'ouverture de pointe de la canule est de 5 % à 60 %.

7. Canule d'aspiration chirurgicale selon l'une quelconque des revendications 1 à 6, dans laquelle la structure d'aspiration souple (2) comprend une structure d'aspiration jetable pouvant être raccordée à et retirée d'un corps de canule d'aspiration (1).

8. Canule d'aspiration chirurgicale selon l'une quelconque des revendications 1 à 7, dans laquelle une longueur allant de l'ouverture de pointe (3) à une extrémité de pointe de la structure d'aspiration souple (2) représente de 0,2 fois à 10 fois un diamètre de l'ouverture de pointe (3).

9. Unité d'extrémité de pointe de canule d'aspiration chirurgicale comprenant une structure d'aspiration (2), l'unité d'extrémité de pointe de canule d'aspiration chirurgicale pouvant former la canule d'aspiration chirurgicale selon les revendications 1 à 8 en étant raccordée au corps de canule d'aspiration (1).

10. Unité d'extrémité de pointe de canule d'aspiration chirurgicale selon la revendication 9, comprenant une structure d'aspiration (2) et une ouverture de pointe de canule (3),
dans laquelle la structure d'aspiration (2) est agencée au niveau d'une paroi tubulaire d'ouverture de pointe de canule.

11. Instrument de traitement chirurgical comprenant :
la canule d'aspiration chirurgicale selon l'une quelconque des revendications 1 à 8 ; et
des moyens d'incision et de coagulation et/ou des moyens de nettoyage.
